# EUROPEAN PATENT APPLICATION

(11) **EP 2 301 610 A2**
(43) Date of publication of application: **30.03.2011**
(21) Application number: 10178229.0
(22) Date of filing: 22.09.2010
(51) Int. Cl.: A61M 5/158, A61M 25/06, A61M 5/00

(54) **Blood vessel access instrument with vessel entry indicator**

(30) Priority: 23.09.2009 US 564976
(71) Applicant: Tyco Healthcare Group LP, Mansfield, MA 02048 (US)
(72) Inventor: Holley, Steven A, West Roxbury, MA 02132 (US); Stewart, Bradley M, Trevor, WI 53179 (US)
(74) Representative: Gray, James

(57) **Abstract**

A surgical instrument for administering fluids and having a vessel entry indicator to confirm proper placement of the surgical instrument within the vessel includes a vessel access member (100) adapted for accessing a blood vessel and having insertion and trailing ends (110). The access member defines a primary lumen (118) for administering fluids to the blood vessel and a secondary lumen. A pressure detector is in fluid communication with the secondary lumen (120). The pressure detector (124) is adapted to detect pressure associated with a disposition of the insertion end of the access member in the blood vessel. The access member (100) may define a port adjacent the leading end and in fluid communication with the secondary lumen. In one embodiment, the secondary lumen is independent of the primary lumen. The pressure detector (124) may include a float valve responsive to pressure of blood entering from the blood vessel through the port and into the secondary lumen (120). In the alternative, the pressure detector includes a pressure gauge. The pressure gauge is adapted to register pressure associated with a presence of blood entering from the blood vessel through the port and into the secondary lumen.

## Description

### BACKGROUND

### 1. Technical Field

The present disclosure relates to a surgical instrument for accessing a blood vessel, and, in particular, relates to a surgical access instrument incorporating a vessel entry indicator to confirm placement of the access instrument within the blood vessel during administration of fluids.

### 2. Background of Related Art

Surgical access instruments including intravenous (IV) needles or cannulas are employed during the administration of therapeutic fluids into the blood vessel in conjunction with intravenous (IV) procedures. Such IV procedures may include intermittent or continuous IV therapy for the introduction of specialty pharmaceuticals, blood transfusions, chemotherapy regimens, antibiotic therapy, parenteral nutrition, dehydration treatment or the like. During an intravenous procedure, it is imperative that the IV needle remain within the blood vessel and not become inadvertently dislodged or lose access to the blood vessel. For example, during the IV administration of chemotherapy drugs, if the IV needle is dislodged or removed for any reason from the vein, the drugs may enter the subcutaneous tissue and cause damage to the tissue subjected to the drugs.

### SUMMARY

Accordingly, the present disclosure is directed to a surgical instrument for administering fluids and having a vessel entry indicator to confirm proper placement of the surgical instrument within the vessel. The surgical instrument includes a vessel access member adapted for accessing a blood vessel and having insertion and trailing ends. The access member defines a primary lumen for administering fluids to the blood vessel and a secondary lumen. A pressure detector is in fluid communication with the secondary lumen. The pressure detector is adapted to detect pressure associated with a disposition of the insertion end of the access member in the blood vessel. The access member may define a port adjacent the leading end and in fluid communication with the secondary lumen. In one embodiment, the secondary lumen is independent of the primary lumen. The pressure detector may include a float valve responsive to pressure of blood entering from the blood vessel through the port and into the secondary lumen.

In the alternative, the pressure detector includes a pressure gauge. The pressure gauge is adapted to register pressure associated with a presence of blood entering from the blood vessel through the port and into the secondary lumen.

In another embodiment, the secondary lumen is in fluid communication with the primary lumen whereby pressure is detected by the pressure detector upon discontinuance of administering fluids through the primary lumen, to thereby permit blood to pass through the primary lumen and enter the secondary lumen. The pressure detector in this embodiment may be a pressure gauge.

The primary lumen and the secondary lumen may be coaxially arranged about a reference longitudinal axis defined by the access member. Alternatively, the primary lumen and the secondary lumen are offset with respect to a reference longitudinal axis defined by the access member. The access member may include a housing and an elongate member extending from the housing.

A source of therapeutic fluids is adapted to be coupled to the primary lumen.

In another embodiment, the surgical instrument for administering fluids includes a vessel access member adapted for accessing a blood vessel and having insertion and trailing ends. The access member defines a primary lumen for administering fluids to the blood vessel and a secondary lumen. The access member has an inlet port adjacent the leading end and in fluid communication with the secondary lumen to permit blood to pass from the blood vessel to enter the secondary lumen, and a return port displaced from the inlet port and in fluid communication with the primary lumen to permit blood to pass from the secondary lumen to the primary lumen for return with the administering fluids to the blood vessel. A fluid flow detector is in fluid communication with the secondary lumen. The flow detector is adapted to detect passage of blood through the secondary lumen when the insertion end of the access member is disposed within the blood vessel. The fluid flow detector may be a flow gauge. The primary lumen and the secondary lumen may be coaxially arranged about a reference longitudinal axis defined by the access member. Alternatively, the primary lumen and the secondary lumen may be offset with respect to a reference longitudinal axis defined by the access member.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure, wherein:
**FIG. 1** is a side view of a system for administering fluids in accordance with the principles of the present disclosure illustrating the access instrument coupled to a syringe;
**FIG. 2** is a side cross-sectional view of the access instrument of the system illustrating the housing and the elongate member extending from the housing;
**FIG. 3** is a cross-sectional view taken along the lines 3-3 of FIG. 1 illustrating the coaxial arrangement of the primary lumen and the secondary lumens within the elongate member;
**FIG. 4** is a cross-sectional view illustrating an alternate offset arrangement of the primary lumen and the secondary lumen;
**FIG. 5** is a side cross-sectional view of an alternate embodiment of the access instrument; and
**FIG. 6** is a side cross-sectional view of another alternate embodiment of the access instrument.

### DETAILED DESCRIPTION OF PREFERRED EMBODIMENTS

The attached figures illustrate exemplary embodiments of the present disclosure and are referenced to describe the embodiments depicted therein. Hereinafter, the disclosure will be described in detail by explaining the figures wherein like reference numerals represent like parts throughout the several views.

The exemplary embodiments of the apparatus disclosed herein are discussed in terms of performing a therapeutic procedure involving administration of fluids into a blood vessel of the subject. Such therapeutic procedures are inclusive of, but, not limited to, intermittent or continuous IV procedures for the introduction of specialty pharmaceuticals, blood transfusions, chemotherapy regimens, antibiotic therapy, parenteral nutrition, dehydration treatment or the like. However, it is envisioned that the present disclosure may be employed with many applications and related treatments of diseases and body ailments of a subject.

In the following discussion, the term "subject" refers to a human patient or other animal. The term "clinician" refers to a doctor, nurse, or other care provider and may include support personnel. The term "trailing" or "proximal" refers to the portion of the instrument closest to the operator while the term "entry" or "distal" refers to the portion of the instrument remote from the operator.

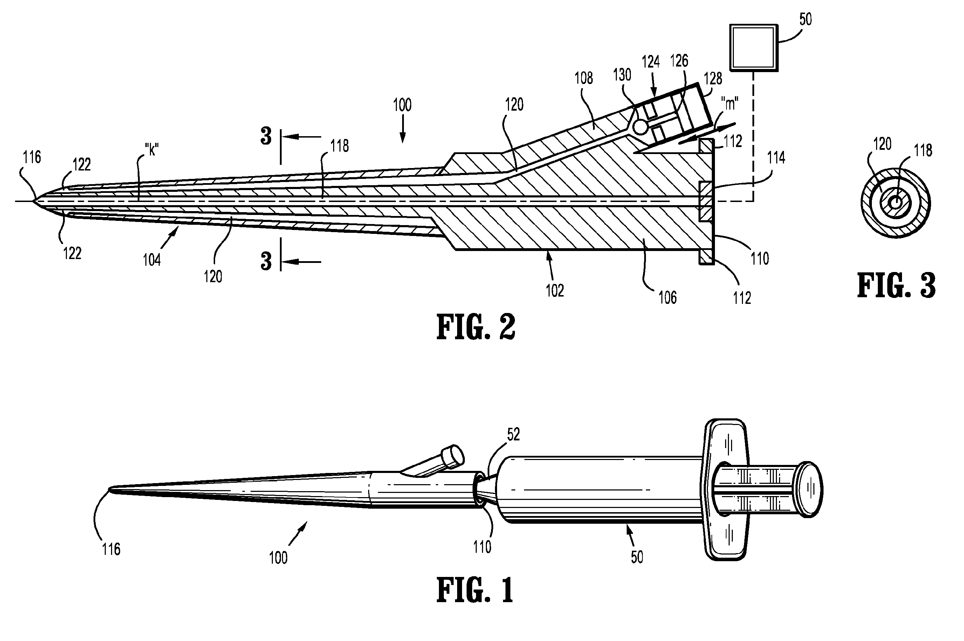

Referring now to **FIG. 1****,** the surgical system in accordance with the principles of the present disclosure is illustrated. System **10** is intended to administer fluids in conjunction with a peripheral intravenous (PIV) procedure. System **10** includes IV fluid source **50** and access instrument **100** which administers the fluids of the IV fluid source **50.** The IV fluid source **50** may be in the form of a syringe as shown in **FIG. 1** or may be an external IV fluid source such as an IV bag , drip or the like. The external IV fluid source **50** is couplable to access instrument **100** through conventional coupling means including, e.g., various commercially available tubings, lines and couplings, which are adapted to connect to access instrument **100.**

Referring now to **FIG. 2****,** in conjunction with **FIG. 1****,** access instrument **100** will be discussed. Access instrument **100** is an access member such as a needle, cannula or catheter adapted to access a blood vessel of the subject, particularly, in connection with peripheral IV procedures where the arm or hands are accessed to deliver IV fluids. Access instrument **100** also may be adapted to access a vein in the leg or foot or, in the case of an infant, the scalp. Access instrument **100** includes housing **102** and elongate member **104** connected to the housing **102** and extending therefrom. Housing **102** is adapted to be grasped by the clinician and may be formed of any suitable metal or polymeric material. Housing **102** may be clear or opaque Housing **102** includes main body **106** and detector leg **108** which extends from the main body **106** in oblique relation to the longitudinal axis "k" of the housing **102.** Main body **106** defines proximal or trailing end 110 which is couplable to IV fluid source **50** through various conventional coupling means. In **FIG. 2****,** IV fluid source **50** is represented schematically and may be an IV bag, drip or the like. Exemplative coupling means for coupling main body **106** to external IV source **50** include luer connectors, bayonet couplings or the like. In **FIG. 2****,** bayonet lugs **112** are shown. As appreciated, the coupling means on the IV line connected to IV fluid source **50** would incorporate corresponding structure to cooperate with the coupling means of main body **106** such as a bayonet female receptor with matching slots for the male bayonet lugs **112.** In the situation where fluid source **50** is a syringe **(****FIG. 1****),** leading end **52** of the syringe **50** may penetrate or pass through internal seal **114** within trailing end **110** of main body **106** to be releasably secured therein. Internal seal **114** is adapted to form a substantial fluid tight seal about syringe leading end **52** and may be a septum seal or zero closure seal fabricated from a suitable elastomeric material.

Elongate member **104** is preferably formed of a suitable biocompatible material such as stainless steel or other polymeric materials. Elongate member **104** may be clear or opaque. The diameter of elongate member **104** may vary, but, typically ranges from a **12-**gauge to a **26** gauge size, e.g., cannula. Elongate member **104** defines leading end **116** which may be adapted to penetrate, incise or pass through tissue to access the vein.

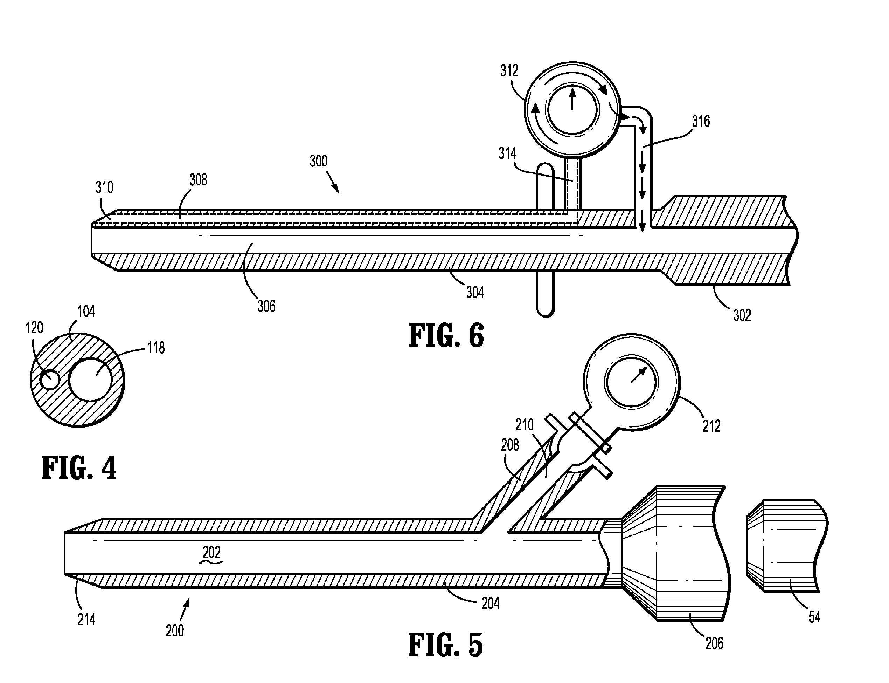

With reference to **FIGS. 1-3****,** access instrument **100** defines primary lumen **118** which extends from trailing end **110** of housing **102** through leading end **116** of elongate member **104.** Primary lumen **118** is in fluid communication with IV fluid source **50** to deliver or administer the IV fluids to the subject's vein. Access instrument **100** further defines secondary lumen **120** which extends through inflow ports **122** adjacent entry end **116** of elongate member **104** through the elongate member **104** and detector leg **108.** Secondary lumen **120** is intended to permit blood to flow from the vein into elongate member **104** and detector leg **108** for reasons to be hereinbelow discussed. Inflow ports **122** may be in diametrical opposed relation and formed in the wall of elongate member **104.** Alternatively, inflow port **122** may be an axial port having an annular configuration. Primary and secondary lumens may be concentrically or coaxially arranged about the longitudinal axis as shown in **FIG. 3****.** In the alternative, primary and secondary lumens **118, 120** may be offset with respect to each other as depicted in **FIG. 4****.**

Referring now to **FIG. 2****,** access instrument **100** further includes pressure detector **124** mounted to detector leg **108** of housing **102** for sensing the pressure within secondary lumen **120.** The pressure is associated with the presence of blood when entry end **116** of elongate member **104** is disposed within vein. Pressure detector **124,** in one embodiment, includes float valve **126** which is mounted within valve housing **128.** Float valve **126** is adapted to reciprocate within housing, in the direction of directional arrows "m", responsive to the presence or lack of presence of blood within secondary lumen **120.** Float valve **126** may be any conventional float valve suitable for this intended purpose including **[Inventor, please provide examples].** In one embodiment, float valve **126** is adapted to reciprocate due to the increased air pressure within secondary lumen due to the presence of blood. In an alternate embodiment, float valve **126** incorporates a floating ball **130** which floats relative to the blood when the blood communicates through the secondary lumen **120** and within valve housing **128.** Various other detectors are envisioned including gauges, transducers, fiber optic sensors, mechanical spring sensors, piezoresistive sensors or transducers, and sensors based upon changes in measured capacitance.

In use, entry end **116** of elongate member **104** is advanced through tissue to access the desired vein. When entry end **116** is positioned in the vein, blood enters through inflow port **122** to pass within secondary lumen **120.** The presence of blood within secondary lumen **120** is detected by float valve **126** which moves in a proximal direction within valve housing **128.** Movement of float valve **126** in the proximal direction is confirmed by the clinician who may view the float valve **126** through a transparent portion of valve housing **128.** Access instrument **100** may then be connected to the fluid source **50** to permit administration of the fluids and commencement of therapy. Throughout the fluid administrating process, the clinician will monitor float valve **126** and determine if the float valve **126** is activated and/or oscillating (corresponding to the rhythm of the pulse) which is indicative of the access instrument **100** being lodged appropriately within the vein.

**FIG. 5** illustrates an alternate embodiment of the present disclosure. In accordance with this embodiment, access instrument **200** includes primary lumen **202** extending the length of elongate member **204** and the length of housing **206.** Access instrument **200** further includes offset detector leg **208** which is arranged in oblique relation to the longitudinal axis of access instrument. Offset leg **208** defines secondary lumen **210** which is in fluid communication with primary lumen **202.** Offset leg **208** further includes pressure gauge **212** mounted thereto. Any suitable commercially available pressure gauge may be employed. Pressure gauge **212** is adapted to monitor pressure within secondary lumen **210** due to the presence of blood within the secondary lumen **210.** In one embodiment, offset leg **208** includes a male/female luer connector which connects to a corresponding connector of pressure gauge **212.** In **FIG. 5****,** IV connector **54** for connecting external IV fluid source **50** to housing **206** is shown.

In use, primary lumen **202** is flushed with a saline. Access instrument **200** is then advanced within the tissue and into the vein with a blood flashback flowing through primary and/or secondary lumens **202, 210** confirming that entry end **214** is within the vein. The presence of blood within primary and/or secondary lumens **202, 210** may be visualized through a transparent region of elongate member **204** or housing **206.** Housing **206** of access instrument **200** is connected to IV fluid connector **54** or a syringe, and administration of fluids through primary lumen **202** is commenced. When it is desired to check that entry end **214** of elongate member **204** is properly positioned within the vein, the infusion or administration of fluids is interrupted. The blood is permitted to communicate through primary lumen **202** and into secondary lumen **210.** Pressure associated with the presence of blood is registered by pressure gauge **212** either through a constant pressure or through an oscillation of pressure (corresponding to the subject's pulse). If no pressure is recorded by pressure gauge **212,** it can be determined that access instrument **200** is not within the vein, thus, prompting the clinician to reposition entry end **214** of the access instrument **200** within the vein.

**FIG. 6** illustrates another alternate embodiment of the present disclosure. Access instrument **300** includes housing **302** and elongate member **304** extending from the housing **302.** Access instrument **300** includes primary lumen **306** extending the length of the access instrument **300** for administration of fluids from a fluid source. Elongate member **304** further includes secondary lumen **308** within the outer wall of elongate member **304** and outlet port **310** in fluid communication with the secondary lumen **308.** Access instrument **300** further includes flow gauge **312** mounted to elongate member **304.** Flow gauge **312** may be any suitable conventional gauge adapted to detect the presence of fluid or detect the flow of fluid. Flow gauge **312** is in fluid communication with primary lumen through inflow tube **314** and outflow tube **316.** Flow gauge **312** may be mounted to housing **302.**

In use, entry end **310** of access instrument **300** is positioned within the vein. Administration of fluids from the fluid source (not shown) through primary lumen **306** is commenced. Concurrently with the administration of fluids, blood enters secondary lumen **308** through entry port **310** and communicates through inflow tube **314** and travels across flow gauge **312.** This flow of blood is registered by flow gauge **312.** The blood thereafter communicates through outflow tube **316** where it is released into primary lumen **300** for return, along with the IV fluids, into the vein of the subject. If no fluid flow is registered by flow gauge **312,** the clinician will determine that access instrument **300** is not properly positioned within the vein, thus, prompting the clinician to act accordingly to reposition the access instrument **300.**

Although the foregoing disclosure has been described in some detail by way of illustration and example, for purposes of clarity or understanding, it will be obvious that certain changes and modifications may be practiced within the scope of the appended claims.

## Claims

1. A surgical instrument for administering fluids, which comprises:
a vessel access member adapted for accessing a blood vessel and having insertion and trailing ends, the access member defining a primary lumen for administering fluids to the blood vessel and a secondary lumen; and
a pressure detector in fluid communication with the secondary lumen, the pressure detector adapted to detect pressure associated with a disposition of the insertion end of the access member in the blood vessel.

2. The surgical instrument according to claim 1 wherein the access member defines a port adjacent the leading end and in fluid communication with the secondary lumen.

3. The surgical instrument according to claim 1 wherein the secondary lumen is independent from the primary lumen.

4. The surgical instrument according to claim 3 wherein the pressure detector includes a float valve, the float valve responsive to pressure of blood entering from the blood vessel through the port and into the secondary lumen.

5. The surgical instrument according to claim 3 wherein the pressure detector includes a pressure gauge, the pressure gauge adapted to register pressure associated with a presence of blood entering from the blood vessel through the port and into the secondary lumen.

6. The surgical instrument according to claim 1 wherein the secondary lumen is in fluid communication with the primary lumen whereby pressure is detected by the pressure detector upon discontinuance of administering fluids through the primary lumen, to thereby permit blood to pass through the primary lumen and enter the secondary lumen.

7. The surgical instrument according to claim 6 wherein the pressure detector is a pressure gauge.

8. The surgical instrument according to claim 1 wherein the primary lumen and the secondary lumen are coaxially arranged about a reference longitudinal axis defined by the access member.

9. The surgical instrument according to claim 1 wherein the access member includes a housing and an elongate member extending from the housing.

10. The surgical instrument according to claim 9 including a source of therapeutic fluids adapted to be coupled to the primary lumen.

11. The surgical instrument according to claim 9 wherein the leading end is associated with the elongate member, the leading end being adapted to penetrate tissue.

12. A surgical instrument for administering fluids, which comprises:
a vessel access member adapted for accessing a blood vessel and having insertion and trailing ends, the access member defining a primary lumen for administering fluids to the blood vessel and a secondary lumen, the access member having an inlet port adjacent the leading end and in fluid communication with the secondary lumen to permit blood to pass from the blood vessel to enter the secondary lumen, and a return port displaced from the inlet port and in fluid communication with the primary lumen to permit blood to pass from the secondary lumen to the primary lumen for return with the administering fluids to the blood vessel; and
a fluid flow detector in fluid communication with the secondary lumen, the flow detector adapted to detect passage of blood through the secondary lumen when the insertion end of the access member is disposed within the blood vessel.

13. The surgical instrument according to claim 12 wherein the fluid flow detector is a flow gauge.

14. The surgical instrument according to claim 12 wherein the primary lumen and the secondary lumen are coaxially arranged about a reference longitudinal axis defined by the access member.

15. The surgical instrument according to claim 1 or 12 wherein the primary lumen and the secondary lumen are offset with respect to a reference longitudinal axis defined by the access member.
